# EUROPEAN PATENT APPLICATION

(11) **EP 4 303 300 A1**
(43) Date of publication of application: **10.01.2024**
(21) Application number: 21928623.4
(22) Date of filing: 23.03.2021
(51) Int. Cl.: C12N 9/26, C12N 15/81, A61K 38/16

(54) **GENE FOR EFFICIENTLY EXPRESSING HYALURONIC ACID HYDROLASE AND EXPRESSION METHOD THEREOF**

(30) Priority: 05.03.2021 CN 202110246672
(71) Applicant: Bloomage Biotechnology Corporation Limited, Jinan, Shandong 250101 (CN)
(72) Inventor: WANG, Hao, Jinan, Shandong 250101 (CN); ZHANG, Tianmeng, Jinan, Shandong 250101 (CN); XU, Rongrong, Jinan, Shandong 250101 (CN); ZHANG, Youheng, Jinan, Shandong 250101 (CN); GUO, Xueping, Jinan, Shandong 250101 (CN); HAO, Jingkun, Jinan, Shandong 250101 (CN)
(74) Representative: Abel & Imray LLP
(86) International application number: PCT/CN2021/082346
(87) International publication number: WO 2022/183542

(57) **Abstract**

Provided is a gene for efficiently expressing hyaluronic acid hydrolase. A nucleotide sequence of the gene is represented by SEQ ID NO: 4. By truncating a segment of a signal peptide sequence at the N-terminus of a full-length hyaluronic acid hydrolase gene, the Pichia pastoris engineering strain of high level expression genetic engineering hyaluronic acid hydrolase is constructed. The enzyme activity of the hyaluronic acid hydrolase in a fermentation broth obtained by high-density fermentation using the constructed Pichia pastoris engineering strain is up to 4.7×10⁵ U/mL.

## Description

### TECHNICALFIELD

The application belongs to the technical field of genetic engineering, and in particular, relates to a gene for efficiently expressing hyaluronic acid hydrolase and an expression method thereof.

### BACKGROUNDART

Hyaluronidase (HAase) is a general term for a class of enzymes that can act on the β-1,3 or β-1,4 glycosidic bond of hyaluronic acid sugar chains to degrade hyaluronic acid, and is widely distributed in nature. Hyaluronidase may be widely applied in clinicas a pharmacologically active substance. For example, it is used in combination with local anesthetics in ophthalmic surgery to speed up the onset of anesthetics.It can promote the diffusion of medicine liquid, exudate or blood stored locally in theeye and the absorption of ocularvitreous opacity, prevent eyeball adhesion after conjunctival chemical burns, and eliminate related inflammatory reactions. Hyaluronic acid filling is an important means of plastic surgery, but hyaluronic acid complications may occur.Hyaluronic acid hydrolase can effectively treat the above adverse reactions as the most effective substance that may be used to hydrolyze hyaluronic acid.Hyaluronidase combined with anti-tumor drugs to assist subcutaneous administration, instead of venipuncture, to improve the medical experience of patients, is currently a hot market application with great potential. In addition, as a tool enzyme, hyaluronidase can efficiently prepare low-molecular-weight hyaluronic acid and hyaluronic acid oligosaccharides, which are more easily absorbed and utilized by the body, and also has huge market prospects in the field of food and cosmetics.

HAases are divided into three types according to differences in their origin, catalytic mechanism and substrate specificity. The first type is hyaluronic acidlyase derived from microorganisms. This type of HAase (EC 4.2.2.1) cleaves the β-1,4glycosidic bond of hyaluronic acid through the β racemization reaction, with the main product being an unsaturated disaccharide molecule 2-acetamido-2-deoxy-3-O-(β-D-gluco-4-enepyranosyluronicacid)-D-glucose. Such hyaluronic acidlyases are mainly derived from *Clostridium, Micrococcus, Streptococcus, Bacillus* and *Streptomyces.* The second type of representative HAase (EC 3.2.1.36) is mainly derived from leech salivary glands and duodenal worms, belongs to endo-β-glucuronidase,and can hydrolyze the β-1,3glycosidic bond of hyaluronic acid with the final product being mainly saturated hyaluronic acidtetrasaccharideand containing a small amount of hyaluronic acidhexasaccharide. This type of HAase has strong substrate specificity and basically does not have hydrolytic and transglycosidic activities on chondroitin sulfate and dermatan sulfate structures. The third type of hyaluronidase is endo-0-N-acetylglucosaminidase (EC 3.2.1.35), is mainly derived from mammalian testis and animal venom, and can hydrolyze β-1,4glycosidic bond of hyaluronic acidwith the final product being mainly hyaluronic acidtetrasaccharide. Such enzymes have a broad substrate spectrum, also have certain catalytic activity on chondroitin sulfate and dermatan sulfate structures, and have transglycosidic activity.

The research on the first type of hyaluronic acidlyase and the second type of endo-β-glucuronidase has been reported in many literatures. Although there are also reports on the third type of endo-β-N-acetylglucosaminidase, the reports mainly focus on the PH20 enzyme derived from mammalian testis, and there are few reports on hyaluronidaseof animal venom. In 2017, KoheiKazuma et al. conducted a complete analysis of the venom components of odontomachusmonticolafor the first time based on transcriptome and peptidomics techniques, and identified a full-length hyaluronic acid hydrolase sequence. At present, there is no relevant report on the recombinant expression of hyaluronic acid hydrolase in odontomachusmonticola.

### SUMMARY

In view of the problems existing in the prior art, the present application provides a gene for efficiently expressing hyaluronic acid hydrolase and an expression method thereof.

Specifically, the present application relates to the following aspects:
1. A gene for efficiently expressing hyaluronic acid hydrolase, having a nucleotide sequence as shown in SEQ ID NO.4.
2. A protein encoded by the gene of item 1, having a sequence as shown in SEQ ID NO.5.
3. A recombinant expression vector comprising the nucleotide sequence of item 1.
4. The recombinant expression vector of item 3, wherein a plasmid backbone is the *Pichia pastoris* vector of pPIC series or pGAP series or pAO815, preferably pPIC9K.
5. A*Pichia pastoris,* comprising the expression vector of item 3 or 4.
6. A method for producing hyaluronic acid hydrolase, comprising:
   producing the hyaluronic acid hydrolase byusing a recombinant *Pichia pastoris* strain comprisingthe gene of item 1 or the *Pichia pastoris* of item 5.
7. The method of item 6, wherein the *Pichia pastoris* is a strain of GS115, KM71 or SMD1168.
8. The method of item 6, wherein the method comprises producingthe hyaluronic acid hydrolasethrough fermentation by BMMY medium and methanolinduction.
9. The method of item 8, wherein conditions of the fermentation are as follows: fermentation temperature is 25°C-30°C, and 0.5%-1% (v/v) methanol is supplemented every 24 hours during fermentation process to induce expression for 96 hours.
10. The method of item 6, wherein the method comprises producingthe hyaluronic acid hydrolase through fermentation by BSM medium and methanolinduction.
11. The method of item 10, wherein the method comprises steps of:
   inoculating*Pichia pastoris* into a seed medium to obtain a seed liquid;
   inoculating the seed liquid into a BSM fermentation medium for fermentation to obtain a fermentation broth containing the hyaluronic acid hydrolase, wherein a fermentation process sequentially comprise four stages: initial fermentation, feeding, starvation culture, and methanol induction.
12. The method of item 11, wherein conditions of the initial fermentation are as follows: fermentation temperature is 25°C-30°C, and pH is 5-7.
13. The method of item 11, wherein a time of starvation culture is 2-3 hours.
14. The method of item 11, wherein the methanol induction is performed at a temperature of 25°C-30°C for 80-120 hours.
15. The method of item 11, wherein the method further comprises purifying the fermentation broth.
16. The method of item 15, wherein the purification is performed by affinity chromatography using nickel column media and gradient elution using 100-500 mM imidazole buffer.
17. Use of the protein of item 2 in the preparation of hyaluronic acid-containing adjuvants, foods and cosmetics.

In the present application, a *Pichia pastoris* engineering strain expressing a high level of genetically engineered hyaluronic acid hydrolase is constructed by truncating a segment of a signal peptide sequence at the N-terminus of the full-length hyaluronic acid hydrolase gene. The enzymatic activity of hyaluronic acid hydrolase in the fermentation broth obtained by high-density fermentation using the constructed*Pichia pastoris* engineering strainisup to 4.7×10⁵U/mL. Therefore, it will be beneficial to further reduce the cost of industrial production of the hyaluronic acid hydrolase of odontomachusmonticola, thereby expanding the application scope of the hyaluronidase in the fields of medicine, chemical industry and food.

### BRIEFDESCRIPTIONOFTHEDRAWINGS

FIG. 1 shows a nucleotide sequence comparisonof the codon-optimized hyaluronic acid hydrolase gene and the wild-type gene.

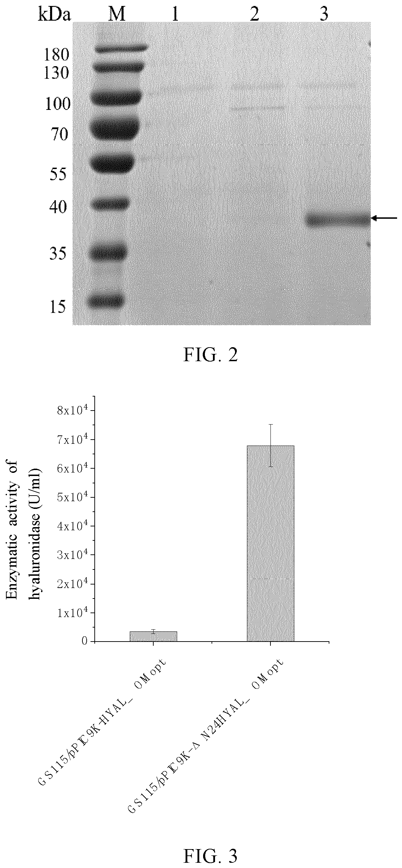

FIG. 2 shows the SDS-PAGE graph of the fermentation supernatant of recombinant hyaluronic acid hydrolase,wherein lane M represents the standard protein with a molecular weight of 180kDa; lane 1 represents the fermentation supernatant of recombinant strain *P. pastoris* GS115/pPIC9K (negative control); lane 2 represents the fermentation supernatant of codon-optimized recombinant strain *P. pastoris* GS115/pPIC9K-HYAL_OM^{opt} (positive control); and lane 3 represents the fermentation supernatant of the recombinant strain *P. pastoris* GS115/pPIC9K-ΔN24HYAL_OM^{opt} constructed in the present application.
FIG.3 shows the crude enzymatic activity of the fermentation supernatant of the codon-optimized recombinant strain *P. pastoris* GS115/pPIC9K-HYAL_OM^{opt} and the recombinant strain *P. pastoris* GS115/pPIC9K-ΔN24HYAL_OM^{opt} constructed in the present application as determined by DNS method.
FIG.4 shows the biomass and enzymatic activity curves of recombinant strain *P. pastoris* GS115/pPIC9K-ΔN24HYAL_OM^{opt} cultured at high density in a 5-L fermenter.
FIG. 5 shows the SDS-PAGE graph of the fermentation supernatant of recombinant strain *P. pastoris* GS115/pPIC9K-ΔN24HYAL_OM^{opt} after gradient elution with imidazole buffer of different concentrations, wherein lane M represents the standard protein with a molecular weight of 180 kDa; and lanes 1-4 represent imidazole concentrations of 100 mM, 200 mM, 300 mM, and 500 mM, respectively.

### DETAILEDDESCRIPTION

The present application will be further described below in conjunction with the examples, and it should be understood that the examples are only used to further illustrate and explain the present application, and are not intended to limit the present application.

Unless otherwise defined, related technical and scientific terms in the presentdescription have the same meaning as those commonly understood by one of ordinary skill in the art. Although methods and materials similar or identical to those described herein may be used in experiments or practical applications, the materials and methods are described below. In case of conflict, the present description, including definitions therein, shall prevail.In addition, the materials, methods and examples are illustrative and not restrictive. The present application is further described below in conjunction with specific examples, but is not intended to limit the scope of the present application.

As used herein, the term "gene" or "coding sequence" refers to a nucleotide sequence in vitro or in vivo that encodes a gene product. In some instances, the gene consists of or essentially consists of coding sequence, that is, a sequence that encodes the gene product. In other instances, the gene comprises additional, non-coding, sequence. For example, the gene may or may not include regions preceding and following the coding region, e.g., 5' untranslated (5' UTR) or "leader" sequences and 3' UTR or "trailer" sequences, as well as intervening sequences (introns) between individual coding segments (exons).

As used herein, the terms "polypeptide", "peptide", and "protein" are used interchangeably herein to mean a polymer of amino acid residues. That is, the description for the polypeptide is also applicable to the peptide and protein, and vice versa. The terms apply to a natural amino acid polymer and an amino acid polymer in which one or more amino acid residues are non-naturally encoded amino acids. As used herein, the terms encompass amino acid chains of any length,

As used herein, the term "polynucleotide" or "nucleotide" means a single-stranded or double-stranded deoxyribonucleotide, deoxyribonucleoside, ribonucleoside or ribonucleotide and polymers thereof. Unless specifically limited, the terms cover nucleic acids containing known analogs of natural nucleotides that have binding properties similar to reference nucleic acids and are metabolized in a manner similar to naturally occurring nucleotides. Unless otherwise limited specifically, the terms also mean oligonucleotide analogs, including PNAs (peptide nucleic acids), DNA analogs used in antisense techniques (phosphorothioate, phosphoramidate, etc.), etc. Unless otherwise specified, a particular nucleic acid sequence also implicitly encompasses its conserved modified variants (including, but not limited to, degenerate codon substitutions) and complementary sequences as well as explicitly specified sequences.

As used herein, the term "vector" is used to describe a nucleic acid molecule that may be engineered to contain a cloned polynucleotide or polynucleotides that may be amplified in a host cell. Vectors include, but are not limited to, nucleic acid molecules that are single-stranded, double-stranded, or partially double-stranded; nucleic acid molecules that comprise one or more free ends, or no free ends (e.g. circular); nucleic acid molecules that comprise DNA, RNA, or both; and other varieties of polynucleotides known in the art. One type of the vector is a "plasmid",which refers to a circular double-stranded DNA loop into which additional DNA segments can be inserted, such as by standard molecular cloning techniques. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operably linked. Such vectors are referred to herein as "expression vectors." Recombinant expression vectors can comprise a nucleic acid of the present application in a form suitable for expression of the nucleic acid in a host cell, which means that the recombinant expression vectors include one or more regulatory elements, which may be selected on basis of the host cells to be used for expression, that is operably linked to the nucleic acid sequence to be expressed.

As used herein, codon optimization refers to a process of modifying a nucleic acid sequence for enhanced expression in the host cells by replacing at least one codon of the natural sequence with codons that are more frequently or most frequently used in the genes of that host cell while maintaining the natural amino acid sequence. Various species exhibit particular bias for certain codons of a particular amino acid. Codon bias (differences in codon usage between organisms) often correlates with the efficiency of translation of messenger RNA (mRNA), which is in turn believed to be especially dependent on the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization.

Codon optimization can be achieved, for example, by transforming nucleotide sequences of one species into the genetic sequence of a different species. Optimal codons help to achieve faster translation rates and high accuracy. As a result of these factors, translational selection is expected to be stronger in highly-expressed genes. However, while optimal codon usage is contemplated herein for expression of disclosed proteins, all possible codons are contemplated for use herein for nucleic acids encoding any disclosed protein.

As used herein, the term "signal peptide" means a short peptide (usually 16-30 amino acids) present at the N-terminus of the majority of newly synthesized proteins that are destined to enter the secretory pathway. It may also be referred to as signal sequence, targeting signal, localization signal, localization sequence, transit peptide, leader sequence or leader peptide. The signal peptide is normally cleaved off from the protein by a signal peptidase.

Specifically, firstly, for the full-length gene sequence of hyaluronic acid hydrolase of odontomachusmonticola, the codon optimization is performed according to the codon preference of *Pichia pastoris* to obtain a codon-optimized hyaluronic acid hydrolase gene, the sequence of which is shown in SEQ ID NO. 1.The protein sequence encoded by it is shown in SEQ ID NO. 2, which is consistent with the amino acid sequence encoded by the hyaluronic acid hydrolase gene of the wild-type odontomachusmonticola. Further, a segment of a signal peptide sequence (SEQ ID NO.3) istruncated at the N-terminus of the codon-optimized hyaluronic acid hydrolase gene to obtain the gene for highly expressing hyaluronic acid hydrolase of the present application, the sequence of which is shown in SEQ ID NO.4,and the protein sequence encoded by it is shown in SEQ ID NO.5. Removing the nucleotide sequence shown in SEQ ID NO.3 from the N-terminus of the codon-optimizedhyaluronic acid hydrolase geneas shown in SEQ ID NO.1 does not cause translation frameshift, hyaluronic acid hydrolase can be expressed and the expression level and/or enzymatic activity of the recombinant hyaluronic acid hydrolasecan be significantly improved. The method for truncating the signal peptide at the N-terminus may be any method known in the art.

Thehyaluronic acid hydrolase of odontomachusmonticolabelongs to the third type of hyaluronidasewhich is endo-β-N-acetylglucosaminidase, and can hydrolyze the β-1,4glycosidic bond of hyaluronic acid to produce the final product having a reducing end of N-acetylglucosamine. In addition, this type of enzyme has a wider substrate spectrumcompared with the second type of hyaluronic acid hydrolase ofleech, and also has a certain catalytic activity on the structures of chondroitin, chondroitin sulfate and dermatan sulfate. The hyaluronic acid hydrolase of odontomachusmonticola can enrich the types of hyaluronic acidhydrolysates, and may have a wider range of applications than hyaluronidase catalysis.

*Pichia pastoris* is a type of yeast inmethylotrophic yeast that can utilize methanol as the sole carbon source and energy source. Like other yeasts, it mainly exists in the form of haploid in the asexual growth phase. When the environmental nutrition is limited, two zygotic haploid cells with different physiological types are often induced to mate and fuse into a diploid. Another biological feature of *Pichiapastoris* is that the alcohol oxidase required for methanol metabolism is sorted into the peroxisome, resulting in regionalization. When glucose is used as carbon source, there are only one or a few small peroxisomes in the thalluses, while when methanol is used as carbon source, peroxisomes account for almost 80% of the entire cell volume, and AOX increases to 35%-40% of total cell protein. Therefore, when the foreign protein gene is inserted by homologous recombination in front of the AOX gene, a large amount of expression can be obtained. At the same time, according to the characteristics of methanol yeast that can form peroxisomes, this system may be used to express some toxic proteins and easily degraded enzymes, and may also be used to study the biogenesis of cell-specific regionalization and its mechanism and function, providing inspiration for similar studies in higher animals.

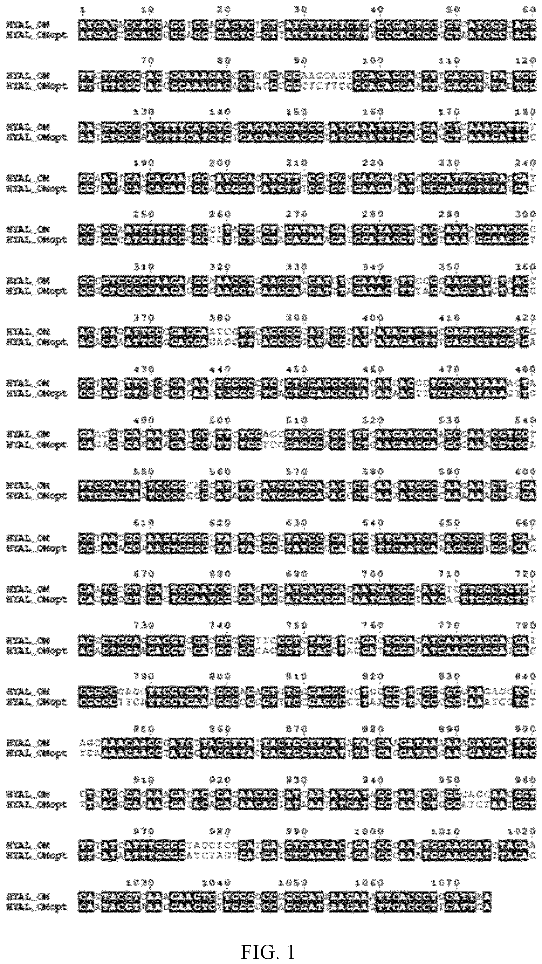

As shown in FIG. 1, the sequence homology between the optimizedhyaluronic acid hydrolase gene of odontomachusmonticolaand the wild-type gene is 73.2%.

The present application also provides a recombinant expression vector comprising the nucleotide sequence of the present application. Further, the backbone of the recombinant expression vector is the *Pichia pastoris* vector of pPIC series or pGAP series or pAO815, preferably the vector pPIC9K. The *Pichia pastoris* vector pPIC series includes pPIC9K, pPIC3.5K, pPICZαA,B,C, pPIC9K-His, pPICZA,B,C, etc, with the alcohol oxidase gene promoter PAOX1 as the promoter. The *Pichia pastoris* vector of pGAP series includes pGAPZαA, pGAPZαB, pGAPZαC, pGAPZA,pGAPZB,pGAPZC, etc., with the glyceraldehyde-3-phosphate dehydrogenase promoter P_{GAP} as the promoter. The vector pPIC9K is a *Pichia pastoris* protein expression vector. The kana resistance gene exists in the pPIC9K vector, which allows the use of kana resistance to screen polyclonal copies in yeast. The rest of the vector is exactly the same as the pPIC9 vector. The host bacteria of *Pichia pastoris* that may be used with the pPIC9K vector include a strain of KM71, GS115 or SMD1168.

The present application also provides a *Pichia pastoris* comprising the above-mentioned expression vector, that is, an expression vector comprising the nucleotide sequence as shown in SEQ ID NO.4.

The present application also provides a method for expressing the hyaluronic acid hydrolase gene of the present application, comprising the step of:
producing the hyaluronic acid hydrolase byusing a recombinant *Pichia pastorisstrain* containing the hyaluronic acid hydrolase gene of the present application or the *Pichia pastorisprovided* by the present application.

In a specific embodiment, the method comprises producing the hyaluronic acid hydrolase through fermentation by BMMY medium and methanolinduction.

The composition of the BMMY medium is yeast extract 10g/L, peptone 20g/L, K₂HPO₄3g/L, KH₂PO₄ 11.8g/L, YNB 3.4g/L, ammonium sulfate 10g/L, biotin 4×10⁻⁴g/L, and methanol 10mL/L.

In a preferred embodiment, conditions of the fermentation are as follows: the fermentation temperature is 25°C-30°C, and 0.5%-1% (v/v) methanol is supplemented every 24 hours during fermentation process toinduceexpression for 96 hours.

In a more preferred embodiment, the recombinant *Pichia pastorisP. pastoris* GS115/pPIC9K-ΔN24HYAL_OM^{opt} containing the hyaluronic acid hydrolase gene of the present application is used as a production strainto produce the hyaluronic acid hydrolase by fermentation. The specific fermentation conditions are as follows:
*P. pastoris* GS115/pPIC9K-ΔN24HYAL_OM^{opt} is inoculated in 40 mL of YPD medium, cultured at 30°C for 24 hours, and then the seed culture solution is inoculated into 40 mL of BMGY medium at 10% of the inoculumamount for 24 hours of culture. The thalluses arecentrifuged, washed, and transferred to the induction medium BMMY, and 1% (v/v) methanol is supplemented every 24 hours to induceexpression for 96 hours.

The present application also provides another method for expressing the hyaluronic acid hydrolase gene of the present application, comprising the following steps:
*Pichia pastoris* is inoculated into a seed medium to obtain a seed liquid;
the seed liquid is inoculated into the BSM fermentation medium for fermentation to obtain a fermentation broth containing hyaluronic acid hydrolase, wherein the fermentation process sequentially includes four stages: initial fermentation, feeding, starvation culture, and methanol induction.

The composition of BSM medium is glycerol 40g/L, K₂SO₄18g/L, KOH 4.13g/L, 85%H₃PO₄ 26.7mL/L, CaSO₄·2H₂O 0.93g/L, MgSO₄·7H₂O 14.9g/L, and filter-sterilized PTM14.4mL/L.The formula of PTM1 is: CuSO₄·5H₂O 6g/L, KI 0.09g/L, MnSO₄·H₂O 3g/L, H₃BO₃ 0.02g/L, MoNa₂O₄·2H₂O 0.2g/L, CoCl₂·6H₂O 0.92g/L, ZnCl₂ 20g/L, FeSO₄·7H₂O 65g/L, biotin 0.2g/L, and H₂SO₄ 5.0mL.

In the first stage of initial fermentation, the fermentation temperature is 25°C-30°C, and the pH is 5-7. The pH is controlled by automatic addition of ammonia water.

In the secondstage of feeding, after the glycerol in the BSM medium is exhausted, the batch feeding stage is entered, and 50% (v/v) glycerol (containing 12 mL/L PTM1) is supplemented by exponential feeding, while the rotation speed is set to couple with dissolved oxygen DO. In a preferred embodiment, the feeding rate is 13.5 mL/h/L, 16.2 mL/h/L, 19.2 mL/h/L, 22.8 mL/h/L, 27.2 mL/h/L and 32.4 mL/h/L respectively for the first 6 hours, and is set to 30 mL/h/Lfor the following 6hours.

In the third stageof starvation culture, starvation culture is performed for 2-3 hours until the residual glycerol is exhausted, wherein the starvation culture is to study the effect of a single factor on the growth of microorganisms or the production of metabolites, the cells that have been cultured to a certain stage are separated from the medium, and cultured for a certain period of time without the nutrient matrix to exhaust endogenous nutrients accumulated in the cells.

In the fourth stage of methanol induction, the methanol induction is performed at a temperature of 25°C-30°Cfor 80-120 hours.

In a preferred embodiment, pure methanol containing 12 mL/L PTM1 is fed with the final concentration maintained at 1.8% (v/v), while the fermentation temperature is adjusted to 25°C, the rotation speed is increased to 1000 rpm, and the feeding rate of the methanol and the final concentration of the methanol in the medium are controlled online in real time by a methanol detector.

The method of the present applicationfurther includes further purifying the above-mentioned fermentation broth.

In a preferred embodiment, the hyaluronic acid hydrolase protein is purified by affinity chromatography using nickel column media and gradient elution using 100-500 mM imidazole buffer.

The enzymatic activity characterization of the hyaluronic acid hydrolase produced by fermentation is determined by the plate transparent circle method, and the enzymatic activity of the fermentation broth supernatant is determined by the 3,5-dinitrosalicylic acid method (DNS).

The definition of activity unit (U) of hyaluronic acid hydrolase is as follows: the amount of enzyme required to release 1 µg glucose reducing equivalent of reducing sugar from hyaluronic acid sugar chain per hour at pH 5.5 and 38°C.

DNS reducing sugar assay: the hyaluronic acid hydrolase of odontomachusmonticola hydrolyzes the β-1,4glycosidic bond of hyaluronic acid to produce a reducing sugar product with a hydroxyl groupat the reducing end. The amount of reducing sugar product relative to glucose reducing equivalents produced by hydrolysis of hyaluronic acidis measured by the DNS reducing sugar method, to calculate the catalytic activity of hyaluronic acid hydrolase.

The present application also provides use of the protein as shown in SEQ ID NO. 5 in the preparation of hyaluronic acid-containing adjuvants, foods and cosmetics.

### Example

### Example 1 Construction ofan expression system containing codon-optimized hyaluronic acid hydrolase gene (HYAL_OMopt)

Based on the full-length gene sequence of hyaluronic acid hydrolase ofodontomachusmonticola(Genbank: FX985505.1) published in the NCBI database, the codon optimization was carried out according to the codon preference of *Pichia pastoris.* As shown in FIG. 1, the optimized gene sequence is shown in SEQ ID NO. 1, and has73.2% homology with the sequence of wild-type gene. The amino acid sequence encoded by the optimized gene is shown in SEQ ID NO. 2, which is consistent with the amino acid sequence encoded by the wild-type gene. The codon-optimized sequence of hyaluronic acid hydrolase of odontomachusmonticola was entrusted to Nanjing GenScript Biotechnology Co., Ltd. for whole gene synthesis, and cloned into the expression vector of *Pichia pastoris* pPIC9K between the EcoRI and NotIenzyme cutting sites to obtain recombinant expression Vector pPIC9K-HYAL_OM^{opt}. The recombinant sequence was correct after DNA sequencing and comparison. The recombinant expression plasmid pPIC9K-HYAL_OM^{opt} was linearized by SalIfast cutting enzyme and then electro-transformed into *P*.*pastoris* GS115 expressing host cells. The recombinant transformants were screened by Geneticin G418 to obtain high-copy recombinant *Pichia pastorisP. pastoris* GS115/pPIC9K-HYAL_OM^{opt} .

### Example 2 Construction of an expression system containing the gene for highly expressinghyaluronic acid hydrolase (ΔN24HYAL_OM^{opt})

Using the above-mentioned pPIC9K-HYAL_OM^{opt} recombinant expression vector as a template, primers were designed. A segment of the signal peptide sequence at the N-terminus of the full-length hyaluronic acid hydrolase gene was truncatedto obtain a genetically engineered hyaluronic acid hydrolase gene fragment.

The primer sequences are as follows:
Upstream primer F:
   5'-CCGGAATTCATGAAGACACTACGCGGCTC-3' (SEQ ID NO. 6);
Downstream primer R:
   5'-ATTTGCGGCCGCTCAATGATGATGATGGTGGTGATGAAGGGTGAACTTCTT-3' (SEQ ID NO. 7);

It should be noted that the GAATTC sequence in the upstream primer is the introduced EcoRI restriction enzyme cutting site, the GCGGCCGC sequence in the downstream primer is the introduced NotI restriction enzyme site, and the reverse complementary sequence of the ATGATGATGATGGTGGTG sequence in the downstream primer encodes 6×His-tag tags.

The amplified genetically engineered hyaluronic acid hydrolase gene fragmentwas subjected to EcoRI and NotI double enzyme digestion, thencloned into the linear expression vector pPIC9K treated with the same digestion, and transformed into *E.coil* TOP10. The recombinant expression plasmid pPIC9K-ΔN24HYAL_OM^{opt} was obtained under the premise of no frameshifting, and the recombinant sequence was correct after DNA sequencing and comparison. The recombinant plasmid was linearized by restriction endonuclease SalI and then electro-transformed into *P. pastoris* GS115 cells. The recombinant transformants were screened by Geneticin G418 to obtain the recombinant strain *P. pastoris* GS115/pPIC9K-ΔN24HYAL_OM^{opt} with high-copy hyaluronic acid hydrolase gene.

### Example 3 Heterologous expression of hyaluronic acid hydrolase by recombinant Pichia pastoris

The obtained recombinant engineering strainP *pastoris* GS115/pPIC9K-HYAL_OM^{opt} and *P*. *pastoris*GS115/pPIC9K-ΔN24HYAL_OM^{opt} were respectively subjected to shake flask fermentation culture. The fermentation steps were as follows:a single clone was picked, inoculated in 40 mL of YPD medium (yeast extract 10 g/L, peptone 20 g/L, glucose 20 g/L), and cultured at 30°C,200 rpm for 24 hours. It was transferred to 40 mL of initial expression medium BMGY (yeast extract 10 g/L, peptone 20 g/L, K₂HPO₄ 3 g/L, KH₂PO₄11.8 g/L, YNB 3.4 g/L, ammonium sulfate 10 g/L, biotin 4×10⁻⁴g/L, glycerol 10mL/L) at 10% inoculum amount, and cultured at 30°C,200rpm for 24 hours. The thalluses were collected by centrifugation, washed with normal saline, and then changed to 40 mL of induction expression medium BMMY (yeast extract 10 g/L, peptone 20 g/L, K₂HPO₄3 g/L, KH₂PO₄11.8 g/L, YNB 3.4 g/L, ammonium sulfate 10g/L, biotin 4×10⁻⁴g/L, methanol 10mL/L) for culture, pure methanol was added to the medium every 24 hours to a final concentration of 1.0% (v/v ) to induce expression for 96 hours.

The fermentation supernatant was detected by SDS-PAGE protein electrophoresis, and the results are shown in FIG. 2. Compared with the recombinant strain in lane 2 (the recombinant strain*P*. *pastoris* GS115/pPIC9K-HYAL_OM^{opt} containing the full-length hyaluronic acid hydrolase gene sequence), there is an obvious protein band (indicated by the arrow) at the theoretical molecular weight of about 39kDa, indicating that the recombinase has achieved efficient secretion expression after truncation of its signal peptide sequence.

### Example 4Enzymatic activity detection of shake flask fermentation supernatant of the recombinant strain

The reducing sugar equivalent produced by the hydrolysis of hyaluronic acid was determined by DNS method, and the activity of hyaluronic acid hydrolase was calculated by standard curve with analytically pure glucose. 1 mL of reaction system: 10 µL of fermentation supernatant (the boil-inactivated fermentation supernatant with an equal volume was used as the blank) and 190 µLof 50 mM citric acid buffer (pH 5.5) were added to 800 µL of 2 mg/mL hyaluronic acid substrate solution, and incubated at 38°C for 15min. After the reaction, the mixture was immediately placed in a boiling water bath for 2min to inactivate the enzyme to terminate the reaction. The treated 1mL reaction solution was added to 2mL DNS (potassium sodium tartrate tetrahydrate 248g/L, 3,5-dinitrosalicylic acid 6.3g/L, 2M NaOH 250mL/L, phenol 5.136g/L, sodium sulfite 5g/L) solution. The obtained mix was shakedto mix well, subjected for boiling water bath together with the glucose standard curve sample for 10min, and then cooled to room temperature in an ice bath. 7mL of deionized water was added and shaking was performed to mix well. The absorbance was measuredat 540nm, and converted into the amount of reducing sugar produced by the reaction. The crude enzymatic activity of the fermentation supernatant of the recombinant engineering strain was calculated according to the glucose standard curve. The results are shown in FIG. 3, the enzymatic activity of the fermentation supernatant of the recombinant strain *P. pastoris* GS115/pPIC9K-HYAL_OM^{opt} constructed by the present application is 3547.88 U/mL, and the enzymatic activity of the fermentation supernatant of the recombinant strain *P. pastoris* GS115/pPIC9K-ΔN24HYAL_OM^{opt} reaches 67970.04U/mL.

### Example 5 High-density culture of recombinant strains in a5-L fermenter

The recombinant strain *P. pastoris* GS115/pPIC9K-ΔN24HYAL_OM^{opt}was subjected to high density culture in a 5-L fermentor. The single colony on the YPD plate was transferred to 50 mL of YPD liquid medium, cultured at 220 rpm, 30°C for 24 hours, and the culture solution was inoculated into 200 mL of BMGY medium at 10%, and cultured at 30°C, 220 rpm for 24 hours.The 200 mL seed solution cultured for 24 hours was added into a 5-L fermenter containing 2 L BSMfermentation medium (glycerol 40g/L, K₂SO₄18g/L, KOH 4.13g/L, 85%H₃PO₄ 26.7mL/L, CaSO₄·2H₂O 0.93gAL, MgSO₄·7H₂O 14.9g/L, filter-sterilized PTM14.4mL/L;the formula of PTM1 is: CuSO₄·5H₂O 6g/L, KI 0.09g/L, MnSO₄·H₂O 3g/L, H₃BO₃ 0.02g/L, MoNa₂O₄·2H₂O 0.2g/L, CoCl₂·6H₂O 0.92g/L, ZnCl₂ 20g/L, FeSO₄·7H₂O 65g/L, biotin 0.2g/L, H₂SO₄ 5.0mL). The initial fermentation parameters were set as temperature of 30°C, pH of 5.5, aeration volume of 2.0vvm and rotation speed of 500rpm.During the fermentation process, the pH was controlled at 5.5 by automatically adding ammonia water.After the glycerol in the BSM medium was exhausted, the batch feeding stage was entered. 50% (v/v) glycerol (containing 12 mL/L PTM1) was supplemented by exponential feeding, while the rotation speed was set to couple with dissolved oxygen DO. The feeding rate was 13.5 mL/h/L, 16.2 mL/h/L, 19.2 mL/h/L, 22.8 mL/h/L, 27.2 mL/h/L and 32.4 mL/h/L respectively for the first 6 hours, and is set to 30 mL/h/Lfor the following 6 hours. After the feeding, the stage of starvation culture was entered. The starvation culture was performed for 2-3 hours until the residual glycerolwas exhausted. The stage of methanol induction was entered. The pure methanol containing 12mL/L PTM1 was fed with the final concentration maintained at 1.8% (v/v), while the fermentation temperature was adjusted to 25°C, the rotation speed was increased to 1000rpm, and the feeding rate of the methanol and the final concentration of the methanol in the medium were controlled online in real time by a methanol detector. After entering the stage of methanol induction, samples were taken at regular intervals to detect the enzymatic activity of the fermentation supernatant.

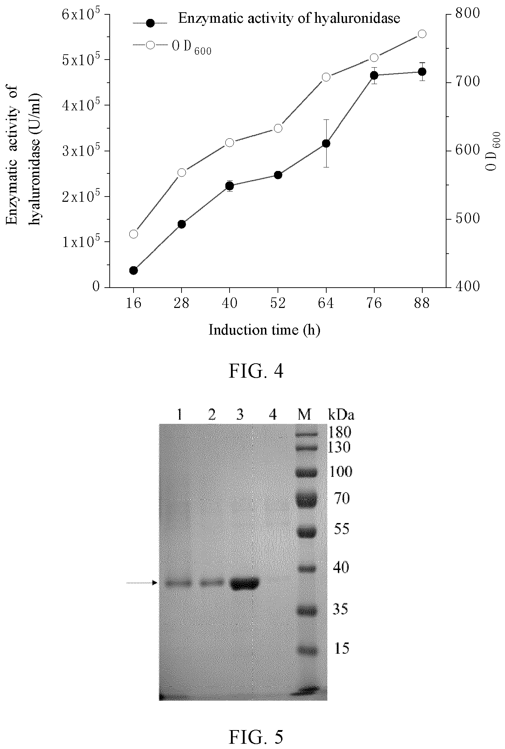

The detection results of enzymatic activity are shown in FIG. 4. When methanol induction is performed for 88 hours, the highest enzymatic activity of hyaluronic acid hydrolase in the fermentation supernatant is 4.7×10⁵U/mL. The enzymatic activity or protein expression level obtained by high-density fermentation is 6.97 times that of shake flask fermentation, making it possible for industrial production.

### Example 6 Purification of genetically engineered hyaluronic acid hydrolase

Six polyhistidine purification tags were introduced at the C-terminus of the genetically engineered hyaluronic acid hydrolase, and could specifically bind to nickel ions to purify the protein. Firstly, the nickel column media was filled into an affinity chromatography column, and then the nickel column was activated with 5 column volumes of binding buffer(20 mM sodium phosphate, 0.5 NaCl, 20 mM imidazole, pH 6.0). An appropriate amount of ultrafiltration concentrated fermentation supernatantfiltered through 0.22 µmwas added, impurities wereeluted with 5 column volumes of binding buffer, and then the target protein was gradient eluted with 10 column volumes of elution buffer having100mM, 200mM, 300mM, and 500 mM imidazole, respectively. The eluent was verified by SDS-PAGE, and the results are shown in FIG. 5. Lane 3 is the protein band by elution with 300 mM imidazole buffer, with the highest protein concentration and a single band. The obtained pure protein of genetically engineered hyaluronic acid hydrolase has normal hyaluronic acid hydrolase activity by enzymatic activity assay.

### Sequence listing

SEQ ID NO. 1: 
atgatcccacccgcacgtgactcgcttatgtttgtctttgcgactgcggtaatcgctagt 60
tttttcggtagcgcaaagacactacgcggctcttccccacagcaattcgacgtatactgg 120
aatgtgccaactttcatgtgtcacaagcacggtatgaaatttgaagagctgaaagatttc 180
ggtatacaccagaacgcaatggatatgtttcgcggcgaagaaattgcgattctttatgac 240
cctggcatgtttcccgcccttctagtagataaagatggatacgtcactaaacggaacggt 300
ggggtgccgcaagaggggaacctcaaggaacatttagaaacctttagaaagcatctgacg 360
acacaaattccggacgagagctttagcgggataggaatcatagactttgagagttggaga 420
 
ccgattttcaggcagaactgggcgtcactcgagccctataaaactttgtccataaagttg 480

gagagggaaaaacacccattttggtcggaggcagctgtgaagaaggaggccaaacgtcga 540

ttcgagaaatccgggcgaatatttatggaggaaaccctcaaaatggccaaaaaactaaga 600

ccgaaagcaaagtggggctattatgggtatccccactgtttcaatcaaacccctggacag 660

cagtcggttcactgcaatcggcaaacgatgatggaaaatgacggtatgagttggctgttt 720

acactcgaagacgttcatgctcccagcgtttacctacgattggaaatcaaggaggatgac 780

cggccttcattcgtgaaaggccgggtttccgaggccttaaggttagccgctaaatcgtct 840

tcaaaacaacgtatcctaccttactactggttcatttatcaggataagaaggatgagttc 900

ttaacggaaaaggatacacaaaacactataaatatgatcgctaatctgggatctaatggt 960

ttcataatttggggatctagtgacgatgtcaacacggaacgcaaatgcaaggatttacag 1020

caatacgtaaaggaagtcttgggcccagcgattaagaagttcacccttcattga 1074
SEQ ID NO. 2: 
Met Ile Pro Pro Ala Arg Asp SerLeu Met Phe Val Phe Ala Thr Ala
1               5                   10                  15
Val Ile Ala SerPhePheGlySer Ala Lys ThrLeuArgGlySerSer
            20                  25                  30
Pro GlnGlnPhe Asp Val Tyr TrpAsn Val Pro ThrPhe Met Cys His
        35                  40                  45
Lys His Gly Met Lys PheGluGluLeu Lys Asp PheGly Ile His Gln
    50                  55                  60
Asn Ala Met Asp Met PheArgGlyGluGlu Ile Ala Ile Leu Tyr Asp
65                  70                  75                  80
Pro Gly Met Phe Pro Ala LeuLeu Val Asp Lys Asp Gly Tyr Val Thr
                85                  90                  95
Lys ArgAsnGlyGly Val Pro GlnGluGlyAsnLeu Lys Glu His Leu
            100                 105                 110
GluThrPheArg Lys His LeuThrThrGln Ile Pro Asp GluSerPhe
        115                 120                 125
SerGly Ile Gly Ile Ile Asp PheGluSerTrpArg Pro Ile PheArg
 
130 135 140
GlnAsnTrp Ala SerLeuGlu Pro Tyr Lys ThrLeuSer Ile Lys Leu
145 150 155 160
GluArgGlu Lys His Pro PheTrpSerGlu Ala Ala Val Lys LysGlu
165 170 175
Ala Lys ArgArgPheGlu Lys SerGlyArg Ile Phe Met GluGluThr
180 185 190
Leu Lys Met Ala Lys LysLeuArg Pro Lys Ala Lys TrpGly Tyr Tyr
195 200 205
Gly Tyr Pro His CysPheAsnGlnThr Pro GlyGlnGlnSer Val His
210 215 220
CysAsnArgGlnThr Met MetGluAsn Asp Gly Met SerTrpLeuPhe
225 230 235 240
ThrLeuGlu Asp Val His Ala Pro Ser Val Tyr LeuArgLeuGlu Ile
245 250 255
Lys Glu Asp AspArg Pro SerPhe Val Lys GlyArg Val SerGlu Ala
260 265 270
LeuArgLeu Ala Ala Lys SerSerSer Lys GlnArg Ile Leu Pro Tyr
275 280 285
Tyr TrpPhe Ile Tyr Gln Asp Lys Lys Asp GluPheLeuThrGlu Lys
290 295 300
Asp ThrGlnAsnThr Ile Asn Met Ile Ala AsnLeuGlySerAsnGly
305 310 315 320
Phe Ile IleTrpGlySerSer Asp Asp Val AsnThrGluArg Lys Cys
325 330 335
Lys Asp LeuGlnGln Tyr Val Lys Glu Val LeuGly Pro Ala Ile Lys
340 345 350
Lys PheThrLeu His
355
SEQ ID NO. 3 
atcccacccgcacgtgactcgcttatgtttgtctttgcgactgcggtaatcgctagtttt 60
ttcggtagcg ca 72
SEQ ID NO. 4 
atgaagacactacgcggctcttccccacagcaattcgacgtatactggaatgtgccaact 60
ttcatgtgtcacaagcacggtatgaaatttgaagagctgaaagatttcggtatacaccag 120
aacgcaatggatatgtttcgcggcgaagaaattgcgattctttatgaccctggcatgttt 180
cccgcccttctagtagataaagatggatacgtcactaaacggaacggtggggtgccgcaa 240
gaggggaacctcaaggaacatttagaaacctttagaaagcatctgacgacacaaattccg 300
gacgagagctttagcgggataggaatcatagactttgagagttggagaccgattttcagg 360
cagaactgggcgtcactcgagccctataaaactttgtccataaagttggagagggaaaaa 420
cacccattttggtcggaggcagctgtgaagaaggaggccaaacgtcgattcgagaaatcc 480
gggcgaatatttatggaggaaaccctcaaaatggccaaaaaactaagaccgaaagcaaag 540
tggggctattatgggtatccccactgtttcaatcaaacccctggacagcagtcggttcac 600
tgcaatcggcaaacgatgatggaaaatgacggtatgagttggctgtttacactcgaagac 660
gttcatgctcccagcgtttacctacgattggaaatcaaggaggatgaccggccttcattc 720
gtgaaaggccgggtttccgaggccttaaggttagccgctaaatcgtcttcaaaacaacgt 780
atcctaccttactactggttcatttatcaggataagaaggatgagttcttaacggaaaag 840
gatacacaaaacactataaatatgatcgctaatctgggatctaatggtttcataatttgg 900
ggatctagtgacgatgtcaacacggaacgcaaatgcaaggatttacagcaatacgtaaag 960
gaagtcttgggcccagcgattaagaagttcacccttcatcaccaccatcatcatcattga 1020
SEQ ID NO. 5: 
Met Lys ThrLeuArgGlySerSer Pro GlnGlnPhe Asp Val Tyr Trp
1 5 10 15
Asn Val Pro ThrPhe Met Cys His Lys His Gly Met Lys PheGluGlu
20 25 30
Leu Lys Asp PheGly Ile His GlnAsn Ala Met Asp Met PheArgGly
35 40 45
 
GluGlu Ile Ala Ile Leu Tyr Asp Pro Gly Met Phe Pro Ala LeuLeu
50 55 60
Val Asp Lys Asp Gly Tyr Val Thr Lys ArgAsnGlyGly Val Pro Gln
65 70 75 80
GluGlyAsnLeu Lys Glu His LeuGluThrPheArg Lys His LeuThr
85 90 95
ThrGln Ile Pro Asp GluSerPheSerGly Ile Gly Ile Ile Asp Phe
100 105 110
GluSerTrpArg Pro Ile PheArgGlnAsnTrp Ala SerLeuGlu Pro
115 120 125
Tyr Lys ThrLeuSer Ile Lys LeuGluArgGlu Lys His Pro PheTrp
130 135 140
SerGlu Ala Ala Val Lys LysGlu Ala Lys ArgArgPheGlu Lys Ser
145 150 155 160
GlyArg Ile Phe Met GluGluThrLeu Lys Met Ala Lys LysLeuArg
165 170 175
Pro Lys Ala Lys TrpGly Tyr TyrGly Tyr Pro His CysPheAsnGln
180 185 190
Thr Pro GlyGlnGlnSer Val His CysAsnArgGlnThr Met MetGlu
195 200 205
Asn Asp Gly Met SerTrpLeuPheThrLeuGlu Asp Val His Ala Pro
210 215 220
Ser Val Tyr LeuArgLeuGlu Ile Lys Glu Asp AspArg Pro SerPhe
225 230 235 240
Val Lys GlyArg Val SerGlu Ala LeuArgLeu Ala Ala Lys SerSer
245 250 255
Ser Lys GlnArg Ile Leu Pro Tyr TyrTrpPhe Ile Tyr Gln Asp Lys
260 265 270
Lys Asp GluPheLeuThrGlu Lys Asp ThrGlnAsnThr Ile Asn Met
275 280 285
 
Ile Ala AsnLeuGlySerAsnGlyPhe Ile IleTrpGlySerSer Asp
290 295 300
Asp Val AsnThrGluArg Lys Cys Lys Asp LeuGlnGln Tyr Val Lys
305 310 315 320
Glu Val LeuGly Pro Ala Ile Lys LysPheThrLeu His HisHisHis
325 330 335
His HisHis
SEQ ID NO. 6:
   CCGGAATTCATGAAGACACTACGCGGCTC
SEQ ID NO. 7:
   ATTTGCGGCCGCTCAATGATGATGATGGTGGTGATGAA GGGTGAACTTCTT

## Claims

1. A gene for efficiently expressing hyaluronic acid hydrolase, having a nucleotide sequence as shown in SEQ ID NO.4.

2. A protein encoded by the gene of claim 1, having a sequence as shown in SEQ ID NO.5.

3. A recombinant expression vector, comprising the nucleotide sequence of claim 1.

4. The recombinant expression vector of claim 3, wherein a plasmid backbone is *a Pichia pastoris* vector of pPIC series, or pGAP series, or pAO815, preferably pPIC9K.

5. *\ Pichiapastoris,* comprising the expression vector of claim 3 or 4.

6. Amethod for producing hyaluronic acid hydrolase, comprising the step of:
producing the hyaluronic acid hydrolase by using a recombinant *Pichia pastoris* strain comprising the gene of claim 1 or the *Pichia pastoris* of claim 5.

7. The method of claim 6, wherein the *Pichia pastoris* is a strain of GS115, KM71 or SMD1168.

8. The method of claim 6, wherein the method comprises producingthe hyaluronic acid hydrolase through fermentation by BMMY medium and methanolinduction.

9. The method of claim 8, wherein conditions of the fermentation are as follows: fermentation temperature is 25°C-30°C, and 0.5%-1% (v/v) methanol is supplemented every 24 hours during fermentation processtoinduce expression for 96 hours.

10. The method of claim 6, wherein the method comprises producingthe hyaluronic acid hydrolase through fermentation by BSM medium and methanolinduction.

11. The method of claim 10, wherein themethodcomprises steps of:
inoculating*Pichia pastoris* into a seed medium to obtain a seed liquid;
inoculating the seed liquid into a BSM fermentation medium for fermentation to obtain a fermentation broth containing the hyaluronic acid hydrolase, wherein a fermentation process sequentially comprise four stages: initial fermentation, feeding, starvation culture, and methanol induction.

12. The method of claim 11, wherein conditions of the initial fermentation are as follows: fermentation temperature is 25°C-30°C, and pH is 5-7.

13. The method of claim 11, wherein a time of the starvation culture is 2-3hours.

14. The method of claim 11,wherein the methanol induction is performed at a temperature of 25°C-30°C for 80-120 hours.

15. The method of claim 11,wherein the method further comprises purifying the fermentation broth.

16. The method of claim 15,wherein the purification is performed by affinity chromatography using nickel column media and gradient elution using 100-500 mM imidazole buffer.

17. Use of the protein of claim 2 in the preparation of hyaluronic acid-containing adjuvants, foods and cosmetics.
